# EUROPEAN PATENT APPLICATION

(11) **EP 1 384 450 A1**
(43) Date of publication of application: **28.01.2004**
(21) Application number: 03016379.4
(22) Date of filing: 19.07.2003
(51) Int. Cl.: A61F 2/00

(54) **Flat implant for use in surgery**

(30) Priority: 21.07.2002 US 201440
(71) Applicant: AESCULAP AG, 78532 Tuttlingen (DE)
(72) Inventor: Goldmann, Helmut, 78532 Tuttlingen (DE); Bertholdt, Günther, 73092 Heiningen (DE); Langanke, Dennis, 78532 Tuttlingen (DE); Weis, Christine, 78532 Tuttlingen (DE)
(74) Representative: Patentanwälte Ruff, Wilhelm, Beier, Dauster & Partner

(57) **Abstract**

A flat implant for use in surgery is described. The implant includes a flexible fabric comprising two sides and having on one side a substantially closed surface and on the other side a three-dimensional microstructure premitting a growing in of cells. The implant can be more particularly used for the treatment of wall defects in body cavities, such as abdominal wall defects.

## Description

The present invention relates to a flat implant for use in surgery and a process for its production.

Hernia is a frequently encountered illness. It generally consists of a passage of organs or organ parts out of the natural body cavity through a preformed or acquired gap. Among external hernias, where the hernial sac is always surrounded by the peritoneum, the most frequently encountered forms are groin, umbilical and incisional hernias. The reason for hernias occurring are in particular muscle or connective tissue weakness in conjunction with overstressing, age-caused atonia, congenital weakness of the abdominal wall or inadequate cicatrization following a body section (incisional hernia).

In most cases effective treatment by surgery is possible, where the hernia content is transferred back from the hernial sac into the abdomen and the hernial opening is closed. This closure of the hernial opening normally takes place by a suture.

However, this surgical procedure suffers from the disadvantage that in up to 20 % of the cases a further hernia occurs, i. e. the so-called hernial relapse.

Due to this unsatisfactory relapse rate following conventional hernia operations, in modern hernia surgery increasing use is being made of artificial strengthening materials for the reconstruction of the abdominal wall. Polypropylene and also polyester nets play an important part.
Although the use of such nets has clearly led to a marked decrease in the relapse rate, such implants are not unproblematical due to possible infections or fistula formations and in particular due to the soft tissue adhesion risk.

The hitherto known implants consist of open textile structures, which aid an adhesion of cells and also a growing through of cells. This is advantageous, because it forms a firm connection between the implant and the abdominal wall and thus ensures the desired support function. However, it can lead to difficultly removable cicatrizations in the abdominal cavity with hardening effects, detrimental action on the intestine and internal organs and the associated complaints on the part of the patient.

Flat, textile implants are also known, which are sealed with an impregnating agent. However, problems then arise in fixing to the abdominal wall, particularly when spending a long time in the body.

The problem is to make available an implant for use in surgery, which overcomes the difficulties of prior art implants, which is easy and inexpensive to manufacture and which is usable by employing conventional surgical methods.

This problem is solved by a flat implant according to claim 1. The implant according to the invention permits a good anchoring of body cells on the structured side facing the abdominal wall and prevents an undesired cicatrization with organs and body parts on the substantially closed side facing the abdominal cavity.

For use in surgery the invention provides a flat implant made of a flexible fabric comprising two sides and having on one side a substantially closed surface and on the other side a three-dimensional microstructure permitting a growing in of cells. In particular the substantially closed surface comprises micropores, the micropores being so small that they permit an exchange of materials, but substantially prevent the growing in of cells, and wherein an additional adhesion prevention is provided by a bioabsorbable component which has a sealing effect. Preferalby, the additional adhesion prevention is provided on the outer surface of the substantially closed surface.

Preferably the substantially closed surface is smooth. The substantially closed surface is formed by at least one surface layer connected to the flexible fabric. In a preferred embodiment of the invention the surface layer is provided by a coating of said fabric.

According to the invention, the three-dimensional microstructure can have back-engageable points for the growing in of cells. Advantageously, the flexible fabric is formed by a porous, flexible structural material, particularly a flexible support, and the three-dimensional microstructure is formed by the exposed surface structure of the structural material. Examples of porous, flexible structural materials are open-cell structural foam or a lattice structure.

Advantageously, the implant is formed by at least one synthetic polymer material. For example, the fabric of the implant according to the invention is formed from polypropylene (PP), polyester, polytetrafluororethylene (PTFE) and/or polyester and PTFE. In a currently preferred embodiment preference is given to a flat PP based implant, according to the invention. Polypropylene is particularly preferred as an implant material as it promotes the growth of cells into the implant structure. It is also possible to use resorbable materials such as polylactides, polyglycolides and copolymers thereof, if a resorbability or partial resorbability is desired.

The additional bioabsorbable component is advantageously selected from biocompatible natural or synthetic compounds, preferably polyhydroxy compounds. Preferably the bioabsorbable component has film forming characteristics. According to the invention the bioabsorbable component can be at least one component selected from the group of polymers and copolymers of organic hydroxyesters, polyglycolide, polylactide, polydioxanone, polyhydroxy butyric acid, polycaprolactone, polytrimethylene carbonate, and polyvinyl alcohol, their derivatives and mixtures thereof. In a currently preferred embodiment of the present invention the bioabsorbable component is polyvinyl alcohol (PVA). Advantageously, the PVA used has a molecular weight from 20000 to 200000 Daltons. Bioabsorbable natural components can be at least one component selected from the group of gelatine and hyaluronic acid. As an alternative, the bioabsorbable component can be a composition of natural and synthetic materials as mentioned above.

The bioabsorbable additive is spread on part or the total of the substantially closed surface of the implant. Due to its sealing effect, the additive will cover residual pores to prevent cell attachment and undesirable tissue adhesion. The bioabsorbability of the additive provides for a temporary adhesion prevention within a period of about two weeks after surgery to promote healing.

In an embodiment of the invention, the flexible structural material can be a textile material, particularly a porous textile support. In a preferred embodiment, the flexible textile support is made of polypropylene. Particularly preferred is an implant, wherein the porous textile support is made of polypropylene monofilaments.

Advantageously, the flexible structural material can be stretchable. The flexible fabric can have two-dimensional stretchability. Furthermore, the flexible structural materials can have elasticity characteristics. The flexible fabric can have two-dimensional elasticity. A support fabric made of polypropylene has characteristics by two-dimensional stretchability and minor resiliency. Preferably the stretchability and strength characteristics of the flat implant are adapted to match the characteristics of the peritoneum in vivo.

Preferably the flexible structural material is a textile fabric. In a particularly preferred embodiment of the implant according to the invention the flexible structural material is a porous textile support. Preferably the flexible textile support can have at least on the side with the three-dimensional microstructure an open textile structure known in connection with vascular implants and which is formed by one or more of textured yarns, float stitches and velour loops. Such structures are known e.g. from patents US 4047252, US 387565, DE 2461370, and US 4517687.

It is possible to process shrinkable fibers and yarns produced therefrom such as well as other fibers and yarns using conventional procedures so as to give textile fabrics. Subsequently as a result of a shrinkage treatment, the shrinkable fibers are shrunk, which in the case of the textile fabrics produced therefrom leads to a compression of the structure. As a result of the intended use of shrinkable fibers and non-shrinkable fibers in combination, it is possible in planned manner to effect modifications to the textile structure.

According to the invention, the fabric can be produced according to a textile method, particularly one of knitting, weaving and braiding. Such procedures are known to the expert, so that there is no need for a detailed explanation here. This permits a simple, inexpensive manufacture according to known proven procedures and using conventional machines and tools.

Advantageously, the textile fabric has open pores sized up to 3 mm in diameter, in particular 0.5 to 2 mm in diameter. In the implant according to the invention, the micropores can have a pore size in the range from 10 µm to 30 µm. The filaments used to prepare the fabric can have a filament diameter from 10 to 500 µm. In particular the textile fabric can have a thickness from 0.4 to 1.0 mm. The textile fabric can have a density (weight area) in the range from 10 to 60 g/m², in particular 20 to 50 g/m². The flat implant according to the invention has a tearing strength of preferably at least 16 N/cm, in particular 16 to 50 N/cm, which is in the range of the strength of the abdominal wall. Thus, there is provided a light-weight, flexible and elastic textile fabric for a surgical implant to meet the requirements in the active life of a patient.

In one embodiment of the invention, the flexible textile fabric, particularly the textile support, can be a woven fabric. In another embodiment of the invention, the flexible textile fabric, particularly the textile support, can be a knitted fabric. In a currently preferred embodiment, the fabric is a warp-knitted fabric. Advantageously the flexible textile fabric has at least on the three-dimensionally structured side exposed fibers or threads serving as an anchoring for cells.

It is possible to use textile fibrous materials such as synthetic monofilaments, multifilament threads or multifilament yarns. Preferably, according to the invention, the fabric can at least partly be formed from multifilament yarns, which can be smooth or structured (textured). In one embodiment of the invention, the yarns can be formed from a single fibrous material type. In another embodiment, the yarns can be formed from several fibrous materials. The yarns can at least partly be formed from highly shrinkable fibrous material. They can also be blends of non-resorbable and resorbable fibrous materials.

The implant according to the invention can in particular be characterized in that one face is formed with a structured surface. According to a preferred embodiment, the structured surface can be in velour form. In a particularly preferred embodiment of the invention, the fabric can be constructed as velour, particularly single velour. According to a further development of the particularly preferred embodiment, the fabric can be constructed as double velour. In particular, the double velour can be constructed with different pile heights on both sides of the fabric.

In a preferred embodiment of the invention, the flexible fabric, particularly the textured support, can be a velour, particularly a double velour, having on the structured side preferably a larger pile height than on the substantially closed side of the implant.

According to the invention, the flat implant can be characterized in that the side opposite to the structured surface is constructed as a smooth surface. In this case smooth means that the surface has no structuring, i. e. as a result of the textile construction, which would permit an anchoring of cell unions. Smooth also means that no fibers of the textile material used project from the surface.

According to the invention, the implant is preferably in the form of a composite structure of two or more different layers. In an embodiment the substantially closed surface can be formed by coating with synthetic material. According to the invention, the coating is preferably constructed as a sprayed coating.

In an embodiment the coating can be a sprayed coating, particularly a sprayed web of a polymer, said polymer being one of soluble and dispersible in a medium, wherein the medium is at least one of non-aqueous, liquid and volatile. In this way it is possible to form a microporous sprayed web, in which initially dissolved sprayed particles are connected with one another and to the surface of the fabric. Advantageously, the substantially closed surface layer is made of polyurethane, particularly uncrosslinked polyurethane. Polylactides and copolymers thereof can also be sprayed from chloroform, instead of polyurethane or mixed therewith, if a resorbability or partial resorbability is desired.

In another embodiment, the substantially closed surface can be formed by the application of a polymer film. Such a polymer film can advantageously be constructed as a microporous film. A suitable material for a polymer film for modifying the implant according to the invention can be a biocompatible material suitable for the intended use as a surgical implant and preferably polypropylene, polyester, polytetrafluoroethylene and/or polyurethane. The application of a synthetic material film for modifying the surface of the textile fabric according to the invention can take place by procedures such as e. g. calender coating or sticking on.

Preferred according to the invention, the surface layer made of polyurethane can have a thickness form 0.1 mm to 0.4 mm. Typically, polyurethane coatings applied to monofilament polypropylene fabrics are thicker than polyurethane coatings applied to multifilament polyester fabrics. Typically, polyurethane coatings are elastic. In an embodiment of the implant according to the invention having multiple surface layers, the layers can have similar elasticity characteristics.

The stretchability and elasticity characteristics of implants for use in abdominal in surgery are important features to allow minimal invasive surgery. Since patients with recurrent hernias often have a weakened connective tissue, new incisions can lead to new hernias and therefore should be kept minimal. So generally minimal invasive techniques, like laparoscopy are advantageous for these patients providing the additional advantage of shorter hospitalization times. To perform laparoscopy, it is necessary to enlarge the space in the abdominal cavity to create an internal operational field. This is generally done by inflating the abdominal cavity with an appropriate gas like carbon dioxide using a pressure of about 15 mm Hg. The inflated state of the abdominal cavity is called pneumoperitoneum. This procedure leads to a stretching of the abdominal wall which depends strongly on the size and form of the individual patient, but will be about 30 to 40 %. The use of an elastic material that can be fixed to the abdominal wall in a stretched configuration provides the advantage to prevent the creation of wrinkles and folds once the pneumoperitoneum is deflated.

A flat implant according to the invention with a polyurethane coating having two-dimensional elasticity, the implant can conform to the contours in the abdominal cavity and reposition to the intended implantation site after release of the abdominal extension by the pneumoperitoneum.

Particularly preferred according to the invention is an implant, wherein the additional adhesion prevention is a PVA coating on top of a polyurethane spray coating. The PVA coating is thin with a thickness up to 0.1 mm.

Preferably a bioabsorbable component can be applied by spray coating. In a particularly preferred embodiment the bioabsorbable component is a polyvinylalcohol spray coating. Such a bioabsorbable component can be effective in adhesion prevention for a period of 10 to 30 days after surgery.

In another embodiment of the invention, the implant can be formed by a three-dimensional web, fibrous materials on the side with the substantially closed surface being closely juxtaposed and on the side with the microstructure form an open union.

The substantially closed surface can be completely tight. If, as preferred, it is microporous, then compared with the porosity of the fabric, it is characterized by a porosity lower by at least a power of 10. In particular, the sprayed coating preferred according to the invention has a deep porosity. Advantageously, the substantially closed surface and in particular the complete implant has an air permeability of 5 to 100 ml of air/cm².min, particularly 25 to 75 ml of air/cm².min, for a pressure difference of 1.2 kPascal. With such a microporosity, an exchange of material in the molecular range is possible. This aids metabolic processes in the vicinity of the implant, aids the supply of essential nutrients and build-up substances, as well as the removal of metabolic waste and harmful substances. This advantageously permits a good compatibility and successful healing. The microporosity of the substantially closed surface is not, however, suitable for the passage or firm and continuous anchoring of large particles such as cells.

The inventive, substantially closed surface leads to a reduction of the structuring of the implant surface on one side. Protuberances and depressions, which e. g. result from the textile fabric manufacturing process, are compensated by the substantially closed, particularly smooth surface. In addition, individual fibers projecting from the textile fabric are enclosed. The closing of the implant surface according to the invention can consequently be considered as a type of surface sealing. The sealing effect of the substantially closed surface is provided by the interaction of both the flexible fabric and the additional application of an adhesion prevention means.

In this way, the substantially closed surface with its limited porosity and small surface structure is unfavourable for an adhesion of cells. The cells find no suitable anchoring points which are necessary for their growth. This essentially prevents a colonization by cells of the substantially closed implant surface.

The implant according to the invention preferably has fraying-proof edges or borders formed by one or more of processing, bonding and welding in a fraying-proof manner.

Advantageously the implant can have a total thickness of approximately 0.1 to 1.2 mm, the thickness of the substantially closed surface being 3 to 15 % thereof. The textile support can have at least one of a woven and knitted basic weave with a thickness of 0.05 to 0.4 mm and an at least unilaterally open structure with a thickness of 0.05 to 0.8 mm.

According to the invention, all the components of the implant are biocompatible and long term-stable. In particular, it can be partly resorbable over the entire surface and in particular the substantially closed surface can comprise completely resorbable material. Thus, following implantation in a physiological medium of the body, there is no surface degradation. The substantially closed surface of the implant consequently maintains its cell-rejecting characteristics, which prevent an adhesion and growing in of cells, such as is provided in accordance with the invention.

Advantageously the implant according to the invention is made from flexible material. Preferably the substantially closed surface can be formed from a solvent-soluble, uncrosslinked polymer. According to a further development, the substantially closed surface can be formed with a rubbery polymer. Preferably, according to the invention, the substantially closed surface can be applied in the form of a solution in a low-boiling solvent to one side of an in particular textile fabric. According to a particularly preferred embodiment of the invention, the substantially closed surface of the flat implant can be formed from uncrosslinked polyurethane.

According to a further development, the implant can contain antimicrobiotic substances, such as e. g. an antibiotic. The administration of antibiotics serves in particular to prevent infection. For prophylaxis and therapy with antibiotics in the surgical field use is e. g. made of cephalosporins such as cephazolin or cephamandol, netilmicin, penicillins such as oxacillin or mezlocillin, tetracycline, metronidazol or aminoglycosides such as gentamicin or neomycin, as well as e. g. rifampicin. In accordance with the particular requirements, experts can select one or more suitable active substances for use. The implant can also contain growth factors.

The present invention also relates to a process for the production of an implant for use in surgery, which comprises the formation of a unilateral, substantially closed surface on one side of a porous, particularly textile fabric for preventing the growing in of cells.

In another embodiment, the invention relates to a process for the production of an implant for use in surgery by forming a substantially closed surface layer and unilaterally, in particular a building-up formation of a three-dimensional microstructure connected thereto.

According to a preferred inventive process, the substantially closed surface can be formed by coating, particularly spray coating of a polymer like polyurethane. The additional adhesion prevention can similarly be formed by coating of a bioresorbable component like polyvinyl alcohol. Preferred coating methods comprise spray coating, dip coating, roll coating, coating application by absorption (capillary suction) and wetting, for example.

In a preferred embodiment of the inventive process, the polyurethane can be sprayed from a solution in low-boiling, organic solvent. Examples of suitable solvents are methylene chloride and chloroform. By evaporating the solvent, the coating film will form. During solvent evaporation fine pores can form in the surface coating, which is advantageous for the implant according to the invention. The thus formed microporosity allows the exchange of materials in the physiological medium. However, the pores are so small that cells are held back.

Also in a preferred embodiment of the inventive process, the polyvinyl alcohol is sprayed from an aqueous solution. The polyvinyl alcohol can advantageously be subject to repeated freeze-thaw cycles to improve its processing characteristics. With an appropriate pretreatment, PVA films can retain elasticity during their life cycle on the flat implant.

According to the invention there is a closing of the surface for preventing the growing in of cells on only one side of the flat implant and on only the surface of the fabric. Advantageously, the three-dimensionally structured surface of the textile fabric is open and suitable for a growing in of cells.

The porous or structured surface is suitable for an adhesion and growing of cells and permits a back-engaging growing in of the cells.

For use in surgery the inventively modified implant can be appropriately sterilized. An appropriate sterilization process can be selected from conventional physical or chemical methods for the inactivation of microorganisms or can be a combination of such methods. One possible sterilization process comprises treatment with ionizing rays, such as e. g. irradiation by β-rays, electron beams, or γ-rays, in the range of 0.1 to 10 Mrad, particularly 0.8 to 2.5 Mrad. Another sterilization method is ethylene oxide treatment.

The invention also relates to the use of an implant in surgery, particularly for the treatment of wall defects in body cavities, particularly abdominal wall defects.

According to the invention a hernial implant is provided comprising a flexible flat three-layered structure including a three-dimensional textile support microstructure permitting a growing in of cells, a substantially closed layer of micropores having a pore size to permit an exchange of materials, but substantially prevent the growing in of cells, and superimposed thereon an additional top layer of a bioabsorbable component which has a sealing effect for adhesion prevention.

To this end the inventively modified implant material can be cut to a desired size and shape. Advantageously, the surgical implant according to the invention is available appropriately packed, ready for use and cut to an appropriate size.

The introduction of the inventive flat implant into the abdominal cavity takes place in such a way that the implant side, whose surface is modified for preventing the growing in of cells, is inserted towards the inside of the abdomen, i. e. facing the intestines. The opposite side of the flat implant, where a growing in of cells is possible, is inserted facing the abdominal wall side. The implant according to the invention is particularly adapted to use with large abdominal wound areas.

Thus, during the healing process, body cells from the abdominal wall can adhere to the implant surface, penetrate the surface structure and over a period of time lead to the cicatrization of the implant with the abdominal wall. This leads to a reliable union between the abdominal wall and the implant, which contributes to the stabilization of the abdominal wall and therefore ensures a successful treatment.

The substantially closed surface for preventing the growing in of cells on the implant abdomen inside is not penetratable by cells growing in from the abdominal wall side.

Further features of the invention can be gathered from the following description of a preferred example in conjunction with the subclaims and drawings. The individual features, both alone and in the form of combinations, can be implemented in an embodiment of the invention. However, the examples only serve to illustrate the invention and do not retrict the latter.

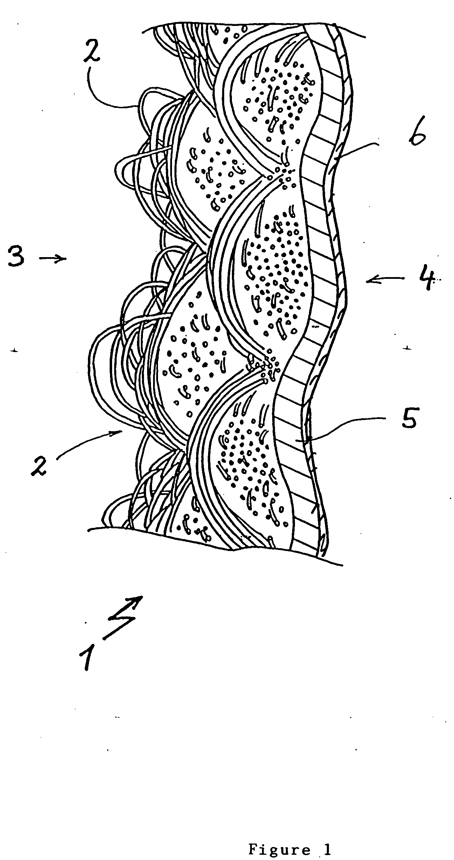

Figure 1 is a diagrammatic section through an implant according to one embodiment of the invention as described in example 1.

### Example 1

A textile fabric 1 is constructed as warp knitting from multifilament polypropylene yarn in the form of a single velour, there being velour loops 2 of textured yarn. The knitted fabric in double velour form can be contructed in similar manner to the knitted fabrics of US patents 4047252 and 4193397. However, it is flat, unlike the tubular warp knitting described therein. It can also be contructed as a single velour. The knitted fabric is porous and flexible. On the velour side 3 it has an open, three-dimensional structured surface, which as a result of the velour loops and texturing of the fibers, has numerous, substantially uniformly surface-distributed back-engagement possibilities for the growing in of body cells. The openings between the yarn loops or the individual fibers are large compared with the size of the body cells. This permits the growing in of a cohesive cell union.

As a result of the texture of the knitted fabric, the opposite side 4 of the knitted fabric 1 is more dense and rather planar. Additionally the knitted fabric 1 has on this side a spray coating 5 of uncrosslinked polyurethane, which is joined to the surface-exposed fibers of the knitted fabric 1 and closes the textile structure on this surface. An additional polyvinyl alcohol sealing layer 6 is applied thereon to provide adhesion prevention. The thickness of the spray coating is approximately 1/10 to 1/20 of the total thickness of the textile fabric and sealing layer, i. e. the total thickness of the flat implant of approximately 0.8 mm.

As a result of spray coating, i. e. the spraying on of a solution of polyurethane in chloroform and an aqueous solution of polyvinyl alcohol, the coating 5 has a structure which can be compared roughly to that of a tight web, is microporous and is also flexible as a result of the elastomeric characteristics of polyurethane and polyvinyl alcohol. Suitable polyurethanes are e. g. known saturated polyesters and polyurethanes. As a result of the sealing layer 5, the textile fabric 1 of velour knitwear is substantianlly closed in the manner of a deep filter on the side directed away from the velour loops 2. The micropores, whose size is roughly of the same order of magnitude as the size of the body cells or smaller, permit an exchange of body fluids for maintaining the metabolism, but do not permit the growing in of cells, or at least not in a form such that it is necessary to fear an adhesion of body parts in the vicinity of the abdominal cavity.

The sealing layer 5 only penetrates insignificantly into the textile surface of the textile support 1, so that the three-dimensional volume of the fabric is available for the growing in of cells from the abdominal wall, whereas the substantially closed sealing layer prevents a rolling in of the knitted fabric and additionally prevents any fraying tendency. If desired, the edges can be rendered completely fraying-proof by welding the polymer fibers. For use in surgery, pieces with the approximate dimensions 8 x 2 cm to 30 x 30 cm are produced. As a function of needs, these pieces can be cut to size prior to implantation.

Animal tests carried out with the implant according to the invention have given good results. On the uncoated, open side of the implants, there was a very good incorporation into the tissue with powerful vascularization in the implant meshes and no repulsion phenomena. As a result of the sealing of the abdominal cavity-side surface, no cicatrization occurred on this side. Even after the implant had been in the abdominal cavity for a long time, a free mobility of the abdominal viscera relative to the implant was ensured.

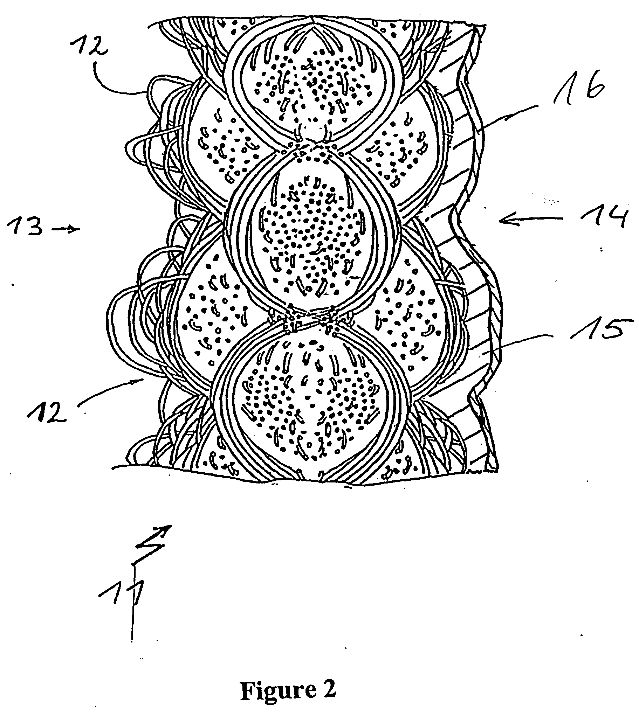

Figure 2 is a diagrammatic section through an implant according to another embodiment of the invention in which the fabric is constructed in double velour form. In particular, figure 2 shows a double velour with velour loops 12 of texturized yarn on both sides. Side 13 of textile fabric 11 has an open three-dimensionally structured surface as also present in figure 1. Side 14 is also a velour side, which is coated with a spray coating 15 of uncrosslinked polyurethane. An outer coating 16 made of a copolymer of glycolide, lactide and trimethylene carbonate provides an initial complete sealing and is resorbable within about 30 days after surgery.

### Example 2

A flat implant made of polypropylene and covered with a polyurethane spray coating is provided with an additional polyvinyl alcohol (PVA) coating by immersion into an PVA solution for adhesion prevention. Thickness of the PVA coating is controlled by process conditions like concentraion of the PVA solution or contacting time.

A very thin nanoscale coating is achieved by dipping the fabric into an about 80 °C hot 1 % PVA solution. While the molecular weight of the PVA applied is not relevant for the coating procedure, the PVA molecular weight is essential for its resorption in vivo. High molecular weight PVA of about 200000 g/mol is preferable for very thin coatings, to provide adaquate attachment of the PVA on the implant surface and adhesion prevention in the patient's abdominal cavity. Thin PVA coatings are transparent and do not affect implant material characteristics.

A thicker PVA coating in the micron range is obtained by immersion of the fabric into PVA hydrogel, optionally repeated, and freezing during 1 to 5 freezing cycles to provide a hydrogel coating of desired strength. There is a significant retardation in resorption time due to physical crosslinking. The higher the molecular weight of the PVA employed, the more stable is the hydrogel formed. Appropriate selection of a composition of low molecular and high molecular weight PVA molecules allows tailoring of PVA resorption characteristics to meet practical requirements. The PVA hydrogel coating is transparent.

### Example 3

Prepared is a flat implant composed of three layers including a warp-knitted support fabric made of polypropylene monofilaments, a microporous spray coating made of linear polyurethane and on top a sealing spray coating made of bioresorbable polyester.

The flat polypropylene support fabric is fixed on a rotating roller of a spray coating apparatus. The spraying procedure comprises a prespraying step and a main spraying step. During the prespraying step, a solution of polyurethane in chloroform having a viscosity of 456.5 mPas and diluted in chloroform in a ratio of 1 : 2 is applied. During the main spraying step, a pure polyurethane soution of controlled viscosity is used. In both cases the spraying nozzles are fixed in an angle of 65° to the roller to perform initial 50 cycles in a distance of 5.0 cm and subsequent 150 cycles in a distance of 24.0 cm. The roller is left to dry for 24 h at room temperature, before the main spraying step of 150 cycles in a distance of 5 cm and 200 cycles in a distance of 24 cm is performed.

The spray coated fabric is dipped in 2-propanol for 10 sec and dryed over night at 21 °C. Subsequently, the fabric is postreated at 50 °C and 98 % rel. humidity in a conditioning chamber for 2 hours. During all the processing steps the fabric remains fixed on the roller. The complete double-layer fabric has an air permeability of 5 to 100 ml air/cm².min, preferably 30 to 70 ml air/cm².min. the thickness of the polyurethane layer alone is from 0.1 to 1 mm, preferably from 0.15 to 0.45 mm.

To apply the third top coating, the roller is again inserted into the spray coating apparatus and a bioresorbable polyester sprayed thereon. The solution includes poly(D,L-lactide) in chloroform having a viscosity of 465.5 mPas. Both spraying nozzles are arranged in an angle of 65° to the roller and there are 100 spraying cycles in a distance of 5.0 cm applied.

The polyester top layer has a thickness from 10 µm to 100 µm. The sealing surface of the third coating has an air permeability in the range of less than 25 ml air/cm².min, and preferably zero air permeability.

A combination of two bioresorbable polyesters will allow control of the resorption time of the third coating. The use of a copolymer of poly(D,L-lactide-co-glycolide) will reduce the resorption time of the top coating as compared to the above poly(D,L-lactide).

### Example 4

Prepared is a flat implant composed of three layers including a warp-knitted support fabric made of polypropylene monofilaments, a permanent microporous spray coating made of linear polyurethane and on top a sealing spray coating made of polyvinyl alcohol.

The application of the intermediate polyurethane coating is according to the procedure as described in example 3.

To apply the third polyvinylalcohol coating, the polyurethane coated polypropylene fabric is immersed into a 3 % aqueous PVA solution. The PVA has a molecular weight of 200000. The fabric is kept immersed in the PVA solution for 1 minute. Subsequently, the PVA is subject to physical crosslinking, whereby the resorption characteristics of the PVA are controlled. The thus coated fabric is subject to three times repeated freeze/thaw cycles, wherein the freezing down to -18 °C is for 20 h followed by 2 hours of thawing at 25 °C. The PVA coating formed is transparent.

## Claims

1. A flat implant for use in surgery, the implant comprising a flexible fabric comprising two sides and having on one side a substantially closed surface and on the other side a three-dimensional microstructure permitting a growing in of cells, wherein the substantially closed surface comprises micropores, the micropores being so small that they permit an exchange of materials, but substantially prevent the growing in of cells, and wherein an additional adhesion prevention is provided by a bioabsorbable component which has a sealing effect.

2. The implant according to claim 1, wherein the substantially closed surface is formed by at least one surface layer connected to the flexible fabric.

3. The implant according to claim 1 or 2, wherein the at least one surface layer is provided by at least one coating of the fabric.

4. The implant according to any of the preceding claims, wherein the three-dimensional microstructure has back-engageable points for the growing in of cells.

5. The implant according to any of the preceding claims, wherein the flexible fabric is formed by a porous, flexible structural material, in particular a flexible support, and the three-dimensional microstructure is formed by the exposed surface structure of the structural material.

6. The implant according to claim 5, wherein the flexible structural material is made of polypropylene.

7. The implant according to claim 5 or 6, wherein the flexible structural material has elasticity characteristics.

8. The implant according to any of the claims 5 to 7, wherein the flexible structural material is a textile fabric, in particular a porous textile support, preferably made of polypropylene monofilaments.

9. The implant according to claim 8, wherein the textile fabric has open pores sized up to 3 mm in diameter, in particular the pores are sized 0.5 to 2 mm.

10. The implant according to claim 8 or 9, wherein the textile fabric has a thickness from 0.4 to 1.0 mm.

11. The implant according to any of claims 5 to 10, wherein the flexible structural material has on at least the side with the three-dimensional microstructure an open textile structure known in connection with vascular implants and which is formed by one or more of textured yarns, float stitches and velour loops.

12. The implant according to any of the preceding claims, wherein the micropores have a pore size in the range from 10 µm to 30 µm.

13. The implant according to any of the preceding claims, wherein the flexible fabric is a woven fabric which at least on the three-dimensionally microstructured side has exposed fibers or threads serving as an anchoring for cells.

14. The implant according to any of the preceding claims, wherein the flexible fabric is a knitted fabric, in particular a warp-knitted fabric, which at least on the three-dimensionally microstructured side has exposed fibers or threads serving as an anchoring for cells.

15. The implant according to any of the preceding claims, wherein the flexible fabric is a velour, in particular a double velour, the double velour having on the structured side preferably a larger pile height than on the substantially closed side of the implant.

16. The implant according to any of the claims 2 to 15, wherein the surface layer is a spray coating, in particular a sprayed web of a polymer, said polymer being one of soluble and dispersible in a medium, wherein the medium is at least one of non-aqueous, liquid and volatile.

17. The implant according to any of the preceding claims, wherein the substantially closed surface layer is made of polyurethane, in particular a polyurethane spray coating applied to one side of the flat implant.

18. The implant according to any of the claim 2 to 17, wherein the surface layer has a thickness of 0.1 mm to 0.4 mm.

19. The implant according to any of the claims 2 to 18, wherein multiple surface layers have similar elasticity characteristics.

20. The implant according to any of the preceding claims, wherein the additional adhesion prevention is provided on the outer surface of the substantially closed surface.

21. The implant according to any of the preceding claims, wherein the bioabsorbable component is at least one component selected from the group of polyvinyl alcohol, polymers and copolymers of organic hydroxyesters.

22. The implant according to any of the preceding claims, wherein the bioabsorbable component is a spray coating.

23. The implant according to any of the preceding claims, wherein the additional adhesion prevention is a PVA coating on top of a polyurethane spray coating.

24. The implant according to any of the preceding claims, wherein the bioabsorbable component is effective in adhesion prevention for a period of 10 to 30 days after surgery.

25. The implant according to any of the preceding claims, wherein the implant without the bioabsorbable material has an air permeability of 5 to 100 ml of air/cm².min for a pressure difference of 1.2 kPascal.

26. The implant according to any of the preceding claims, wherein the implant has fraying proof edges formed by one or more of processing, bonding and welding in a fraying-proof manner.

27. The implant according to any of the preceding claims, wherein the implant has a total thickness of approximately 0.1 to 1.2 mm, the thickness of the substantially closed surface preferably being 3 to 15 % thereof.

28. The implant according to any of the preceding claims, wherein the flexible fabric has at least one of a woven and knitted basic weave with a thickness of 0.05 to 0.4 mm and an at least unilaterally open structure with a thickness of 0.05 to 0.8 mm.

29. The implant according to any of the preceding claims, wherein the substantially closed surface is of completely resorbable material.

30. The implant according to any of the preceding claims, for the treatment of wall defects in body cavities, in particular abdominal wall defects.

31. A hernial implant comprising a flexible flat three-layered structure including a three-dimensional textile support microstructure permitting a growing in of cells, a substantially closed layer of micropores having a pore size to permit an exchange of materials, but substantially prevent the growing in of cells, and superimposed thereon an additional top layer of a bioabsorbable component which has a sealing effect for adhesion prevention.
